Europäisches Patentamt

European Patent Office

Office européen des brevets

(19)

(11) Veröffentlichungsnummer : **0 077 799**
**B1**

(12) **EUROPÄISCHE PATENTSCHRIFT**

(45) Veröffentlichungstag der Patentschrift :
11.11.87

(51) Int. Cl.⁴ : **A 61 N   1/36**

(21) Anmeldenummer : 82901404.2

(22) Anmeldetag : 04.05.82

(86) Internationale Anmeldenummer :
**PCT/DE 82/00098**

(87) Internationale Veröffentlichungsnummer :
**WO/8203780 (11.11.82 Gazette 82/27)**

(54) HERZSCHRITTMACHER.

(30) Priorität : 04.05.81 DE 3118096

(43) Veröffentlichungstag der Anmeldung :
04.05.83 Patentblatt 83/18

(45) Bekanntmachung des Hinweises auf die Patenterteilung : 11.11.87 Patentblatt 87/46

(84) Benannte Vertragsstaaten :
DE FR GB NL

(56) Entgegenhaltungen :
EP-A- 0 001 156
EP-A- 0 015 779
EP-A- 0 032 818
DE-A- 2 951 162
FR-A- 2 443 718
FR-A- 2 492 262
GB-A- 2 026 870
US-A- 4 197 850
IEEE International Solid-State Circuits Conference,
Band 24, Februar 1981, New York (US);
J.A.BERKMAN u.a.: "Biomedical microprocessor
with analog I/O", Seiten 168, 169

(73) Patentinhaber : BIOTRONIK Mess- und Therapiegeräte GmbH & Co Ingenieurbüro Berlin
Sieversufer 8
D-1000 Berlin 47 (DE)

(72) Erfinder : NETTELHORST, Herwig, Frhr., v.
Pössnecker Strasse 30
D-1000 Berlin 45 (DE)
Erfinder : REXHAUSEN, Hermann
Meilinger Strasse 48
D-3200 Hildesheim (DE)
Erfinder : SCHALDACH, Max
Turnstrasse 5
D-8520 Erlangen (DE)
Erfinder : SCHWEIGGERT, Eugen
Selbitzer Strasse 81a
D-1000 Berlin 22 (DE)

(74) Vertreter : Christiansen, Henning, Dipl.-Ing.
CHRISTIANSEN & NINNEMANN Dietrich-Schäfer-
Weg 21
D-1000 Berlin 41 (DE)

## Beschreibung

Die Erfindung betrifft einen Herzschrittmacher der im Oberbegriff des Anspruchs 1 angegebenen Art.

Es ist aus der FR-A-2443718 bei künstlichen Herzschrittmachern bekannt, Zeitgebermittel vorzusehen, welche zu vorgegebenen Zeitpunkten oder nach einem vorgegebenen Programmablauf Impulssteuermittel zur Stimulation des Herzens beeinflussen. Dabei wird durch zusätzliche logische Schaltmittel unter Berücksichtigung von Herzeigenaktionen die zeitliche Lage der Stimulationsimpulse festgelegt bzw. darüber entschieden, ob ein nachfolgender Stimulationsimpuls zu unterdrücken ist.

In diesem Zusammenhang wurde bisher davon ausgegangen, daß sowohl bei der Verwendung diskreter als auch bei der Benutzung programmierbarer Logikschaltungen, diese permanent mit Energie versorgt werden müssen, da sie stets entscheidungsbereit sein müssen.

Weil bei implantierbaren Herzschrittmachern die Energiequelle aber in das Gehäuse einbezogen ist und damit nur einen begrenzten Energievorrat aufweist, andererseits aber die Betriebsdauer des Schrittmachers ohne Wechsel der Energiequelle möglichst groß sein soll, ist es wünschenswert, den Energieverbrauch so gering wie möglich zu halten. Dazu kommt, daß die logischen Entscheidungen und Operationen, welche durchlaufen werden müssen, um festzulegen, ob zu einem vorgegebenen Zeitpunkt ein Stimulationsimpuls abgegeben oder dieser unterdrückt werden soll, mit fortschreitender technischer Entwicklung komplexer werden, so daß auch unter diesem Gesichtspunkt wegen der erforderlichen zusätzlichen Logikschaltmittel die Tendenz zu größerem Stromverbrauch hin geht.

Der im Anspruch 1 angegebenen Erfindung liegt die Aufgabe zugrunde, einen Herzschrittmacher gemäß dem 1. Teil des Anspruchs 1 so zu verbessern, daß dessen Stromverbrauch auch bei der Fähigkeit, komplizierte logische Verknüpfungen auszuführen, so gering ist, daß er entweder längerfristig innerhalb des Körpers des Patienten implantiert verbleiben kann, oder aber bei den derzeit üblichen Implantationszeiträumen in der Lage ist, zusätzliche Entscheidungen aufgrund von aufgenommenen intrakardialen Signalen oder auf vorhandene Störsignale hin zu treffen und mit dieser erhöhten Datenverarbeitungsfähigkeit eine verbesserte Therapie zu ermöglichen.

Die Erfindung geht aus von der Erkenntnis, daß es nicht erforderlich ist, alle Teile der zur Verarbeitung von Logiksignalen vorgesehenen Teile der Schrittmacherschaltung permanent in einem die Signalverarbeitung ermöglichenden Betriebszustand zu halten. Die Logikschaltmittel sind bei der Erfindung deshalb ausschließlich zu Zeiten aktiviert, in denen entweder Signale über die im Herzen plazierten Elektroden aufgenommen werden oder aber ein Zeitpunkt erreicht wird, zu dem ein Stimulationsimpuls an das Herz abgegeben werden soll.

Diejenigen Zeiten, in denen eine Signalaufnahme bzw. -verarbeitung der vom Herzen abgeleiteten Signale gesperrt ist (Refraktärzeiten), stehen bevorzugt zusätzlich für interne Schalt-, Prüf- und Verarbeitungsvorgänge des Schrittmachers sowie für dessen Umprogrammierung zur Verfügung.

Es ist ersichtlich, daß nicht nur eine Aktivierung der zur Verarbeitung von Logiksignalen vorgesehenen Schaltmittel auf von angeschlossenen Eingangsschaltungen ausgehenden Signalen hin erfolgt, sondern, daß eine zusätzliche Zeitgebervorrichtung vorgesehen ist, welche zu denjenigen Zeitpunkten eine Aktivierung bewirkt, die aufgrund der Auswertung vorangehender Ereignisse ermittelt und vorgegeben sind. Die Zeitgebermittel sind also — bei nicht aktivierten weiteren Logikschaltmitteln — mit (aktivierten) Vergleichermitteln und Speichermitteln für Zeitsignale oder Zeiten repräsentierende Zustände einnehmenden Zählern verbunden, wobei die Vergleichermittel ein Signal abgeben, wenn ein den in den Speichermitteln festgehaltenen Signalen entsprechender Zeitpunkt erreicht ist.

Bei einer ersten bevorzugten Ausführungsform der Erfindung werden die Signalverarbeitungsmittel auch durch zusätzliche in den Wartezuständen aufgetretene Ereignisse, wie das Ende von Refraktärzeiten oder das Ende von Störungsereignissen, aktiviert. Da diese Signale fiktiv sind, d. h. nicht mit äußeren Signalereignissen, sondern mit dem Erreichen vorbestimmter Zählerstände von internen zeitgesteuerten Zählern verknüpft sind, werden diese Ereignisse in Form von Zeitmarken bis zur nächsten Beendigung des Wartezustands durch ein äußeres Ereignis festgehalten (oder aber es wird bei Erreichen des entsprechenden Zählerstands der Wartezustand unterbrochen).

Bei einer anderen bevorzugten Ausführungsform der Erfindung sind solche Logikschaltmittel, welche zum Erkennen von äußeren Störsignalen, wie sie beispielsweise durch äußere elektromagnetische Felder oder durch Elektrodenartefakte hervorgerufen werden, permanent aktiviert, da sie wegen ihrer begrenzten Aufgaben verhältnismäßig wenig Strom aufnehmen. Die dauernde Betriebsbereitschaft eines kleinen Teils der Bauelemente zur Verarbeitung von Logiksignalen, welche auf häufig vorkommende Signale ansprechen, benötigt hierbei weniger Energie als das in Funktionsetzen von komplexen Logikschaltmitteln mit einer größeren Signalverarbeitungsfähigkeit in regelmäßigen kurzen Zeitabständen.

Bei der vorgenannten Ausführungsform ist weiterhin vorteilhaft, daß die Schaltmittel, welche zum Erkennen von Störsignalen permanent in Betrieb sind, bei Ausfall der nur zeitweise zu aktivierenden Logikschaltmittel Teile einer Reserveschaltung bilden, welche den Betrieb des Schrittmachers auch bei ausgefallenem komplexen Signalverarbeitungsteil in einem Betriebszustand halten, der eine zur Aufrechterhaltung der vitalen Funktionen des Patienten ausreichende Stimulation gewährleistet. Die dazu erforderlichen Logikschaltmittel entsprechen dem eines bekannten « Demand- » oder festfrequenten Schrittmachers.

Es ist dadurch einerseits bei ordnungsgemäßem Betrieb ein minimaler Stromverbrauch sichergestellt und andererseits bei dem (mit einer größeren Wahrscheinlichkeit behafteten) Ausfall eines Elements der komplexen Logikschaltungen mit größerer Signalverarbeitungsfähigkeit unter nahezu allen Umständen ein Notbetrieb gewährleistet, welcher zwar nicht die vollständige Ausnutzung der mit einem derartigen Schrittmacherkonzept verbundenen Therapiemöglichkeiten bietet, aber dafür die Stimulation bis zu einer unter ärztlicher Aufsicht erfolgenden Maßnahme aufrechterhält.

Besonders vorteilhaft bei der erfindungsgemäßen Lösung ist, daß aufgrund der Taktfrequenz des dauernd im Betrieb befindlichen Zeitgebers ohne zusätzlichen Aufwand eine hohe Auflösung bei der zeitlichen Diskrimination erreicht werden kann, wobei diese Auflösung bei einer Taktfrequenz des Zeitgebers um 1 kHz eine Abtastung mit einem Ein-Millisekunden-Raster ermöglicht.

Die Erfindung läßt sich einerseits in günstiger Weise auf Verarbeitungsschaltungen für Logiksignale anwenden, welche aus diskreten Logikschaltungen bestehen, wobei hierbei die Aktivierung der komplexen Logikschaltungsteile durch zeitweises Einschalten der Stromversorgung für diese Schaltungen bewirkt wird. Bei der Verwendung eines Mikroprozessors für die komplexe Logiksignalverarbeitung andererseits wird dieser in einen « Stand-by »-Zustand gesetzt, in dem sein Stromverbrauch wesentlich herabgesetzt ist, während die permanent in Betrieb befindlichen Zeitgeberschaltungen entweder als Zähl- und Vergleicherschaltung mit herkömmlichen diskreten Schaltmitteln aufgebaut sind oder aber einen Mikroprozessor enthalten, der eine der vorgesehenen Anwendung entsprechende geringere Signalverarbeitungsfähigkeit mit einem entsprechend geringen Stromverbrauch aufweist.

Die Erfindung basiert auf dem Bestreben, eine universelle Schrittmacherstruktur anzugeben, welche Fehlermöglichkeiten bekannter Systeme vermeidet, wobei die Realisierung in einem implantierbaren System möglich sein soll. Das System soll eine weitestgehende Auswertung der aufgrund von vom Herzen abgeleiteten Signalen zur Verfügung stehender Informationen ermöglichen, wobei der Einfluß von Störsignalen gering gehalten ist. Durch die physiologisch korrekte Zuordnung aller die Betriebsarten des Schrittmachers betreffenden Umschaltvorgänge wird eine große Patientensicherheit erreicht.

Vorteilhafte Weiterbildungen sind in den Unteransprüchen angegeben und werden einschließlich der Erfindung anhand des in den Figuren dargestellten Ausführungsbeispiels näher erläutert. Es zeigen :

Figur 1 ein Blockschaltbild der den erfindungsgemäßen Schrittmacher bildenden Baugruppen unter besonderer Berücksichtigung der im Betrieb auftretenden logischen Verknüpfungen,

Figur 2 ein Zeitdiagramm zur Erläuterung des Funktionsablaufs bei dem in Fig. 1 dargestellten Schrittmacher,

Figur 3a Einzelheiten einer zur Erkennung von Störsignalen dienenden Baugruppe und

Figur 3b Einzelheiten einer zur Erkennung von Tachykardien dienenden Baugruppe.

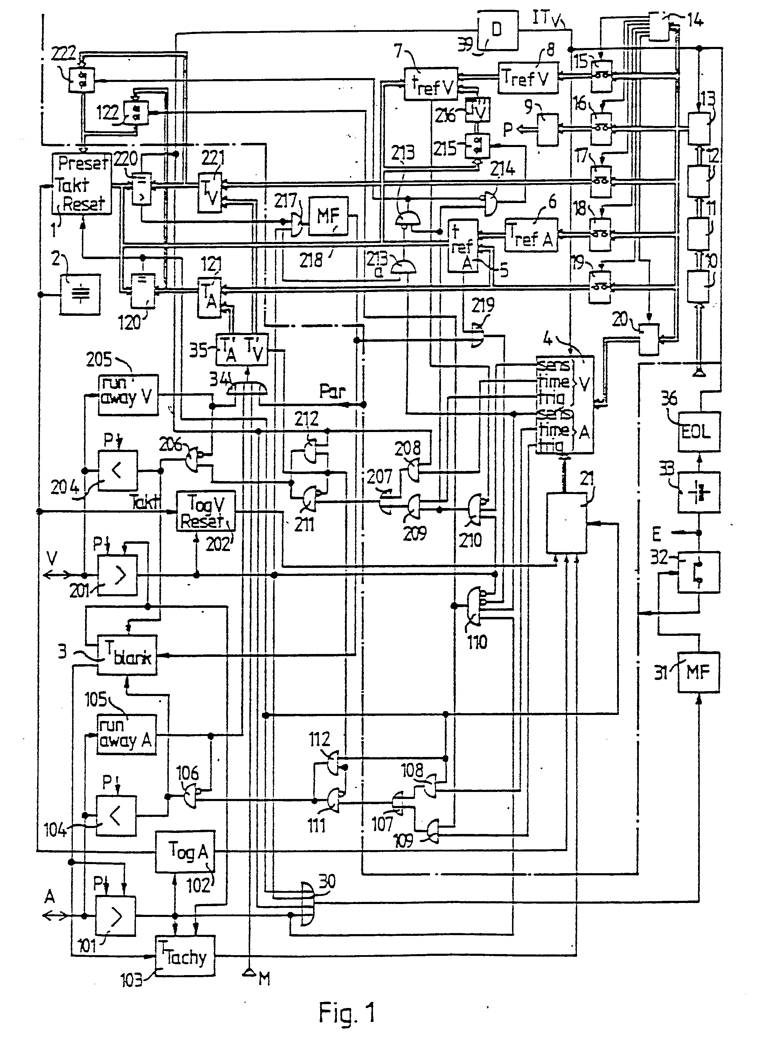

Das in Figur 1 dargestellte in Blockschaltungsform wiedergegebene Prinzipschaltbild des erfindungsgemäßen Herzschrittmachers gibt den Signalfluß zwischen den einzelnen Baugruppen und eine Anzahl von logischen Verknüpfungen wieder. Der verwendeten Schrittmacherstruktur liegt das Konzept eines Schrittmachers zugrunde, das auf grund von Zu- und Abschaltungen von Signalverbindungen in unterschiedlichen Betriebsarten (Modes) benutzt werden kann, wobei die Umschaltung zwischen den einzelnen Betriebsarten (die im Folgenden mit den auf dem Gebiet der Herzschrittmachertechnik eingeführten generischen Codes bezeichnet werden sollen) programmiert und/oder signalabhängig erfolgen kann. In seinen komplexeren Modes paßt sich der Schrittmacher sich dabei auf physiologische Weise dem natürlichen Herzverhalten an.

Die Realisierung des dargestellten Konzepts kann gleichermaßen mittels diskreter oder programmierbarer Logik-Bauelemente erfolgen, wobei im Falle einer hardwaremäßigen Lösung die erforderlichen Verknüpfungen — wie dargestellt — mittels Logik-Gattern und Speichermitteln wie Flip-Flops und Latches erfolgen, während beispielsweise bei einer Mikroprozessorlösung die Logik-Entscheidungen nicht parallel, sondern nach Programm seriell vorgenommen werden. RAM-Speicherplätze übernehmen dabei die Funktion der Speichermittel zum Festhalten von für Betriebszustände charakteristischen Signalen. Einige der dargestellten Funktionsbaugruppen stellen vorteilhafte Weiterbildungen der Erfindung dar.

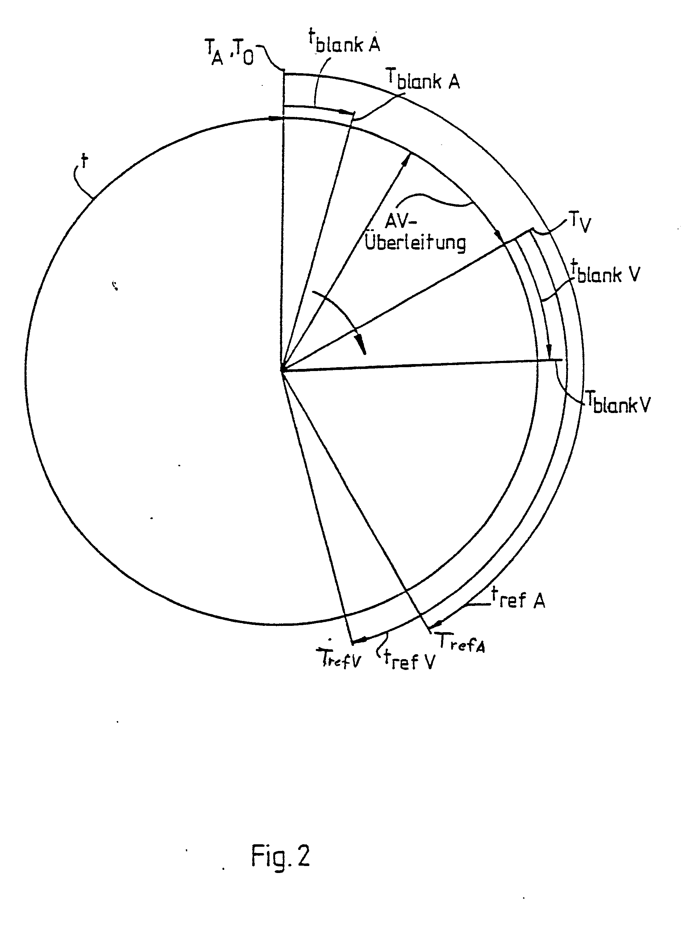

Aus Figur 2 ist der zeitliche Ablauf der Signalverarbeitung und Stimulationsvorgänge ersichtlich, wobei in den übrigen Darstellungen entsprechende Zeitbezeichnungen verwendet wurden, so daß diesbezüglich auf diese Figur stets Bezug genommen werden kann. In der Darstellung fällt der Anfangszeitpunkt $T_0$ zusammen mit dem Zeitpunkt $T_A$, der nach einem Umlauf (entsprechend einem Herzzyklus) erreicht wird und die Aktivität des Atriums charakterisiert. Im Falle einer Stimulation im Ventrikel oder Atrium folgt eine Austastzeit $t_{blankA}$ für die im Atrium plazierte Elektrode, wobei die Dauer dieser Austastzeit jeweils davon abhängig ist, ob im Atrium oder im Ventrikel, d. h. in derselben oder in der jeweils anderen Kammer stimuliert wurde. Das Ende dieser Austastzeit ist mit $T_{blankA}$ bezeichnet.

Zwischen $T_A$ und $T_V$ befindet sich die AV-überleitungszeit, wobei $T_V$ den Zeitpunkt der Aktivität des Ventrikels charakterisiert. An $T_V$ schließt sich entsprechend eine Austastzeit $t_{blankV}$ an, welche im Zeitpunkt $T_{blankV}$ endet.

Die Refraktärzeit des Atriums $t_{refA}$ beginnt mit $T_o$ und endet bei $T_{refA}$, während die Refraktärzeit des Ventrikels bei $T_V$ beginnt und bei $T_{refV}$ endet. Die Zeiten $T_{og}$ und $T_{tachy}$ sind aus dem Diagramm gemäß Figur 2 nicht direkt ersichtlich, da diese durch entsprechende Folgeraten der Herzaktionspotentiale und/oder Störsignale als Triggersignale festgelegt werden.

Bei dem in Figur 1 dargestellten Herzschrittmacher bilden die Anschlüsse A und V Ein- und Ausgänge für Signale, welche vom Herzen aufgenommen werden bzw. in Form von Stimulationsimpulsen an das Herz abgegeben werden. Mit « A » ist dabei der Anschluß für eine im Atrium und mit « V » der Anschluß für eine im Ventrikel fixierte Elektrode bezeichnet.

Die zentrale Steuerung für die zeitliche Zuordnung der abzugebenden Impulse erfolgt über einen Zähler 1, welcher durch von einem Taktgenerator 2 erzeugte Impulse inkrementiert wird und über einen « Reset »-Eingang in eine Ausgangsstellung zurücksetzbar ist. In Abhängigkeit von vom Herzen aufgenommenen Signalen ist dieser Zähler über einen zusätzlichen « Preset »-Eingang auf beliebige Zählerstände in Vorwärtsrichtung — entsprechend einer zeitlichen Voreilung — voreinstellbar. Ein Verändern des Zählerstandes in Rückwärtsrichtung ist nicht notwendig. Nach Erreichen seines Endstandes setzt der nachfolgende Impuls den Zähler auf 0, von wo aus der Zählvorgang fortgesetzt wird. Das Zusammenwirken des Zählers mit den übrigen in Figur 1 dargestellten Bauelementen wird weiter unten näher beschrieben. (Die Übertragung einzelner digitaler Signalimpulse oder analoger Signale ist in den Figuren durch einfache Verbindungen dargestellt, während doppelte Linien die Übertragung vollständiger Datenwörter symbolisieren.)

Die vom Atrium über eine dort fixierte Elektrode aufgenommenen Signale gelangen über den Anschluß A an eine Eingangsstufe 101, welche einen Vorverstärker enthält, der über einen Programmiereingang (zugehöriger Pfeil P) in seinem Verstärkungsfaktor einstellbar ist. Die Eingangsstufe bewirkt gleichzeitig eine Impulsformung in der Weise, daß die vom Atrium aufgenommenen Signale in Form von impulsförmigen Logiksignalen in den nachfolgenden Schaltstufen weiterverarbeitet werden können. Die Darstellung der Logikpegel bei dem wiedergegebenen Ausführungsbeispiel erfolgt in positiver Logik, d. h. das Vorhandensein eines Signals wird mit dem H-Zustand und die Abwesenheit mit dem L-Zustand bezeichnet. Umschaltelemente (Zähler, Mono- und Flip-Flops) werden durch die ansteigenden Vorderflanken der Signalimpulse aktiviert. Weiterhin weist die Eingangsstufe 101 Filtermittel auf, mit einer Filtercharakteristik, die sie vorzugsweise für die aufzunehmenden Nutzsignale durchlässig macht.

Die Eingangsstufe 101 wird außerdem von einer Austastschaltung 3 beeinflußt, welche die Eingangsstufe für vorgegebene Zeiträume $t_{blankA}$ sperrt, wenn ein Stimulationsimpuls an Atrium oder Ventrikel abgegeben wurde. Auf diese Weise wird verhindert, daß ein vom Schrittmacher abgegebener Stimulationsimpuls als herzeigenes Signal verarbeitet wird und das Betriebsverhalten des Schrittmachers fehlerhaft beeinflußt.

Der Eingangsstufe nachgeschaltet ist eine Störerkennungsschaltung 102, welche ein Ausgangssignal abgibt, wenn der Abstand zweier aufeinanderfolgender, von der Eingangsstufe 101 verarbeiteter Signalimpulse einen vorgegebenen zeitlichen Mindestabstand ($T_{og}$), entsprechend einer maximalen Folgefrequenz, unterschreitet. Derartige eine Störung bildende Signale können ihre Ursache sowohl außerhalb (elektromagnetische Einstreuungen oder Wechselströme) als auch innerhalb des Körpers des Patienten (Elektrodenartefakte) haben. Die Störerkennungsschaltung 102 verhindert im Zusammenwirken mit weiter unten zu beschreibenden Mitteln, daß an der Atriumelektrode aufgenommene Impulse, welche die vorgegebene Frequenz überschreiten, zur Weiterverarbeitung zugelassen werden.

Der Ausgang der Eingangsstufe 101 ist weiterhin mit dem Eingang einer Tachykardieerkennungsstufe 103 verbunden, welche entsprechend ein Signal abgibt, wenn die am Ausgang der Stufe 101 erscheinenden Impulse eine vorgegebene Folgezeit unterschreiten. Die « Tachykardierate » $T_{Tachy}$ ist mit ca. 250 ms größer als die als Kriterium für das Vorhandensein eines Störsignals festgelegte Zeitdauer $T_{og}$, welche ca. 100 ms beträgt.

Der Anschluß V, an den eine im Ventrikel zu fixierende Elektrode angeschlossen werden kann, ist mit dem Eingang einer weiteren Eingangsstufe 201 verbunden, die in ihren grundsätzlichen Funktionen der Eingangsstufe 101 für vom Atrium aufgenommene Signale entspricht. Der Verstärkungsfaktor ist über einen zusätzlichen Eingang (zugehöriger Pfeil P) einstellbar. Eine separate Störerkennungsschaltung 202 für die von der im Ventrikel fixierten Elektrode aufgenommenen Störsignale gibt ein Ausgangssignal ab, wenn die vom Ventrikel aufgenommenen Signalimpulse eine höhere als eine vorgegebene Frequenz aufweisen. Die Eingangsstufe 201 wird entsprechend durch von der Austaststufe 3 abgegebene Signale zeitweise gesperrt.

Stimulationsimpulse für das Atrium werden mittels einer Impulsformerschaltung 104 erzeugt, wobei die Amplitude der Stimulationsimpulse über einen zusätzlichen Eingang (zugehöriger Pfeil P) einstellbar ist. Die Impulsformerstufe 104 wird durch kurzzeitige Impulse mit logischem H-Pegel angesteuert und enthält auch die zur Heraufsetzung der Eingangsimpulse auf den zur Stimulation notwendigen Pegel erforderlichen Verstärkungsmittel.

Die von der Impulsformerstufe 104 abgegebenen Impulse gelangen einerseits zum Anschluß A und andererseits zu einer Run-away-Schutzschaltung 105, welche in dem dargestellten Ausführungsbeispiel durch ein Monoflop gebildet wird, dessen Impulsdauer auf die höchste zulässige Stimulationsrate im Atrium abgestimmt ist. Auf einen Ausgangsimpuls von der Impulsformerstufe 104 hin gibt die Schutzschaltung 105 einen Impuls vorgegebener Breite an den invertierenden Eingang eines UND-Gatters 106 ab, welches daraufhin für die Dauer des letztgenannten Impulses hin für an seinen anderen Eingang gelangende Signalimpulse gesperrt ist.

Eine Impulsformerstufe 204 für zum Stimulieren des Ventrikels bestimmte Impulse ist ebenfalls über einen weiteren Eingang (zugehöriger Pfeil P) bezüglich der Amplitude der abgegebenen Impulse beeinflußbar. Eine separate Run-away-Schutzschaltung 205 für den Ventrikel sperrt ein UND-Gatter 206 über dessen invertierenden Eingang, wenn die Folgezeit der den Ventrikel stimulierenden Impulse den vorgegebenen Wert unterschreitet.

Die Zeiten, zu denen Stimulationsimpulse abgegeben werden können (mit $T_A$ und $T_V$ bezeichnet), bestimmt der Zähler 1, wobei jeweils für Atrium und Ventrikel separate Vergleicherschaltungen 120 bzw. 220 vorgesehen sind, welche einen Impuls am Ausgang « = » erzeugen, wenn der Zählerstand des Zählers 1 mit einem vorgegebenen Zählerstand in einem Speicher 121 für den Stimulationszeitpunkt des Atriums bzw. einem entsprechenden Speicher 221 für den Ventrikel übereinstimmt. Die Vergleicherschaltung 220 gibt über einen zusätzlichen Ausgang « > » ein Signal ab, wenn der Zählerstand des Zählers größer ist als der in dem Speicher 221 festgehaltene Wert für $T_V$. Die genannten Zeitmarken $T_A$ und $T_V$ bilden Bezugszeiten für den Betrieb des Schrittmachers und beenden im Demand-Betrieb die sogenannten « Escape-Intervalle » innerhalb denen eine entsprechende Eigenaktion des Herzens die Erzeugung eines Stimulationsimpulses durch den Schrittmacher verhindert.

Die Zeiten $T_A$ und $T_V$ für Atrium und Ventrikel in den Speichern 121 bzw. 221 werden in Form von Zahlenwerten programmiert, welche diejenigen Zählerstände repräsentieren, die der Zähler 1 im Stimulationszeitpunkt erreicht haben muß. Die Zeitzuordnung zu den Zählerständen erfolgt in der Weise, daß die Stimulationszeitpunkte mit einem ausreichend feinen Zeitraster einstellbar sind. Bei einem Zählertakt von beispielsweise 1 kHz weist das zur Verfügung stehende Zeitraster eine Teilung in Millisekunden auf. Die mittels der Speicher 121 bzw. 221 vorgebbaren Zählerstände sind durch weiter unten darzustellende Mittel veränderbar.

Die Eingangsimpulse für die Ausgangsstufe 104, welche das UND-Gatter 106 passieren, stammen vom Ausgang eines ODER-Gatters 107, an dessen Eingänge alternativ die über die Abgabe eines Stimulationsimpulses an das Atrium entscheidenden Signale gelangen. Einer der Eingänge des ODER-Gatters 107 ist mit dem Ausgang eines UND-Gatters 108 verbunden, das ein Ausgangssignal abgibt, wenn die Vergleicherschaltung 120 das Erreichen des Zeitpunktes $T_A$ angibt und damit anzeigt, daß im Demand-Zustand der Vorhofstimulation ein Zeitpunkt erreicht wurde, bei dem wegen fehlender eigener Aktivität des Herzens ein Stimulationsimpuls erforderlich ist. Das UND-Gatter 108 wird über seinen weiteren Eingang mittels eines Signals durchgeschaltet, welches von einem Ausgang « $time_A$ » des Betriebszustandsregister 4 abgegeben wird, wenn eine Impulsabgabe nach Zeitablauf erwünscht ist. Bei freigegebener Signalaufnahme ($sens_V$ bei Block 4) für die entsprechende Kammer erfolgt damit ein « Demand »-Betrieb mit Inhibierung durch herzeigene Impulse, während bei gesperrter Signalaufnahme eine Stimulation zu festen Zeiten erfolgt.

Ein weiteres UND-Gatter 109 wird über einen entsprechenden Ausgang « $trig_A$ » der Schaltung 4 bei durch Herzeigenaktionen im Vorhof getriggerter Signalabgabe an den Vorhof aktiviert. Das Signal zum synchronen Auslösen des Stimulationsimpulses im Vorhof gelangt an den Eingang des UND-Gatters 109 vom Ausgang eines weiteren UND-Gatters 110, das seinerseits durch ein von einem Ausgang « $sens_A$ » der Schaltung 4 an seinen Eingang gelangendes Signal aktiviert wird, wenn die Aufnahme von Signalen aus dem Vorhof bei der gewählten Betriebsweise des Schrittmachers vorgesehen ist.

Es ist ersichtlich, daß für den durch herzeigene Signale getriggerten Betrieb diese Freigabe der Signalaufnahme Voraussetzung ist. An das UND-Gatter 110 gelangt nicht nur das Ausgangssignal der Eingangsstufe 101, sondern an dessen weiteren invertierenden Eingang auch das Ausgangssignal einer Refraktärstufe für den Vorhof 5, welche ein Signal abgibt, wenn für einen Zeitraum $t_{refA}$, der mit dem Erreichen des dem Zeitpunkt $T_A$ entsprechenden Zählerstand durch den Zähler 1 beginnt, die Verarbeitung der im Atrium aufgenommenen Signale zur Auswertung für die Abgabe von Stimulationsimpulsen gesperrt ist. Ein der Refraktärzeit $T_{refA}$ für das Atrium entsprechender Zahlenwert ist in einem Speicher 6 festgehalten und über weiter unten dargestellte Programmiermittel durch äußere Signale veränderbar. Ein vom Ausgang der Schaltung 201 im Falle der Signalaufnahme im Ventrikel gelangendes Signal sperrt über dessen weiteren invertierenden Eingang das UND-Gatter 110, so daß bei gleichzeitig im Vorhof und Ventrikel ankommenden Signalen, das Vorhofsignal gesperrt ist und somit der Ventrikel Priorität hat.

Ein ODER-Gatter 207 und UND-Gatter 208 bis 210 bilden die entsprechenden, über die Abgabe von Stimulationsimpulsen an den Ventrikel bzw. über die Signalaufnahme aus dem Ventrikel entscheidenden Logik-Gatter. Das gewünschte Betriebsverhalten des Schrittmachers läßt sich ebenfalls über drei Ausgänge « $sens_V$ », « $time_V$ » und « $trig_V$ » der Schaltung 4 bestimmen, wobei das an den weiteren Eingang des UND-Gatters 208 gelangende Signal die Stimulation freigibt, wenn der Stand des Zählers 1 dem im Speicher 221 festgehaltenen, dem Zeitpunkt $T_V$ zugeordneten Zählerstand erreicht und somit zu diesem Zeitpunkt ein Ausgangssignal des Vergleichers 220 an das UND-Gatter 208 ausgegeben wird.

Die synchrone Stimulation wird durch das UND-Gatter 209 ausgelöst, dem das Ausgangssignal des UND-Gatters 210, welches die vom Ventrikel aufgenommenen Signalimpulse weiterleitet, zugeführt wird. Das UND-Gatter 210 ist über seinen invertierenden Eingang während der Ventrikel-Refraktärzeit $t_{refV}$ gesperrt, welche mittels eines Vergleichers 7 festgelegt wird, wobei der erreichte Zählwert des Zählers 1 mit dem in dem Speicher 8 festgehaltenen, die Zeitdauer $T_{refV}$ repräsentierenden Zählwert verglichen wird und zu dem Inhalt des Speichers 8 derjenige des Speichers 216, dessen Inhalt $T_V''$ im Normalfall der Zeit $T_V$ entspricht, hinzuaddiert wird. (Unterschiede zwischen der Funktion der Speicher 6 für die Refraktärzeit des Atriums und 7 für die Refraktärzeit des Ventrikels sind aus der folgenden Beschreibung ersichtlich.) Der Inhalt des Speichers 8 ist wie derjenige des Speichers 6 über äußere Programmiermittel veränderbar.

Die Ausgangssignale der UND-Gatter 110 bzw. 210 beeinflussen des weiteren Schalter 122 und 222, mittels denen der Zähler 1 über seinen Preset-Eingang auf die in den Speichern 121 (für den Wert $T_A$) bzw. 221 (für den Wert $T_V$) vorliegenden Werte voreinstellbar ist. Damit wird der Zähler 1 beim Erscheinen eines intrakardialen Signals im Atrium auf den der Zeit $T_A$ bzw. beim Erscheinen eines Signals im Ventrikel auf den der Zeit $T_V$ entsprechenden Zahlenwert vorangesetzt. Auf diese Weise wird der Schrittmacher mit dem Herzverhalten synchronisiert, wenn die UND-Gatter 110 bzw. 210 über ihre entsprechenden Signaleingänge für die Signalaufnahme vom Herzen freigegeben sind. Sind die zuletzt genannten Gatter gesperrt, so erfolgt die Stimulation gegebenenfalls asynchron.

Die Inhalte der Speicher 4, 6 und 8 sowie der eines weiteren Speichers 9, welcher die programmierbaren Verstärkungsfaktoren bzw. Ausgangssignalamplituden repräsentierenden Daten für die Stufen 101 und 201 sowie 104 und 204 (zugeordnete Pfeile P) enthält, sind über die blockschaltungsmäßig dargestellten Programmiermittel zu beeinflussen, welche nachfolgend kurz beschrieben werden sollen :

Die von außerhalb des Schrittmachers über einen geeigneten Nachrichtensignalträger (radiofrequente, optische oder sonstige Signale) in Form von Impulsen aufmodulierten, von einem entsprechenden — nicht dargestellten — Sender abgegebenen Signale, gelangen zu einem Empfänger 10, der die verstärkten Signale einem Dekoder 11 zuleitet, mit dessen Hilfe die zu übertragene ursprüngliche Programmierimpulsfolge wieder hergestellt wird. In einer Prüfstufe 12 wird mittels zusätzlich übertragener redundanter Signale festgestellt, ob die übertragene Information vollständig ist und letztere zutreffendenfalls an einen Steuerbaustein 13 übermittelt, durch den mittels einer ersten Teilinformation eine Auswahlschaltung 14 entsprechend aktiviert wird.

Durch das Ausgangssignal der Schaltung 14 wird eine der Schaltstufen 15 bis 20 betätigt, so daß die zweite übertragene Teilinformation in den mit der aktivierten Stufe der Stufen 15 bis 20 verbundenen Speicher gelangt und dort festgehalten wird. Die bereits erwähnten Speicherstufen 4, 6, 8, 9 sowie 121 und 222 halten die für den Betrieb des Schrittmachers wesentlichen veränderbaren Daten fest, wie sie in der vorangehenden Beschreibung aufgeführt wurden.

Der Speicher 4 ist von zusätzlichen Signalen beeinflußbar, welche aus der Signalverarbeitungsstufe 21 stammen, und eine Umschaltung der Betriebszustände in Abhängigkeit von vom Schrittmacher selbst aus dem Herzen aufgenommenen Signalen bewirkt, wobei die Eingangssignale der Stufe 21 von den Stufen 102 und 202 stammen und im Atrium bzw. im Ventrikel aufgenommene Störsignale repräsentieren, sowie von der Tachykardiestufe 103, welche ein Ausgangssignal abgibt, wenn der Tachykardiezustand festgestellt wurde.

Die von der strichpunktierten Linie umgebenen Bauelemente zur Signalverarbeitung und -speicherung sind jeweils nur zeitweise aktiviert, da die zum Betrieb des Schrittmachers notwendigen logischen Operationen mittels moderner mikroelektronischer Bauelemente in sehr kurzen Zeiten ausgeführt werden können, so daß deren permanenter Betrieb einen unnötigen Energieverbrauch zur Folge haben würde. Die außerhalb der genannten Linie befindlichen Bauelemente, die entweder direkt mit den im Herzen fixierten Elektroden verbunden sind und damit die Signalaufnahme vom Herzen kontrollieren, bzw. diejenigen Mittel, welche die Zeitgeber- und Zeitvergleichermittel bilden, sind dauernd aktiviert, da durch letztere bestimmt wird, zu welchen Zeitpunkten logische Operationen auszuführen sind.

Die Aktivierung der innerhalb der strichpunktierten Linie befindlichen Bauelemente erfolgt durch die von den Vergleichermitteln 120 bzw. 220 abgegebenen Signale, wenn ein vorgegebener Zeitpunkt erreicht wurde, oder aber durch die Ausgangssignale der Eingangsstufen 101 bzw. 201, wenn nämlich vom herzen (außerhalb der jeweiligen Refraktärzeit) ein Signal aufgenommen wurde. Die Zusammenfassung der genannten Signale erfolgt über ein ODER-Gatter 30, welches über ein Monoflop 31 einen Schalter 32 betätigt, der die in der gewählten Darstellung innerhalb der strichpunktierten Linie angeordneten Bauelemente mit einer Energiequelle 33 verbindet, wobei dieses « Verbinden » mit einer Energiequelle in jedem Heraufsetzen der Energieversorgung, also auch in einem Umschalten der betreffenden Bauelemente von einem Bereitschafts-(Stand-by-) Zustand in einen Betriebszustand bestehen kann.

Die Impulsdauer des Monoflops 31 ist dabei so bemessen, daß zum Ausführen der notwendigen Schaltvorgänge ein ausreichend langer Zeitraum zur Verfügung steht. Diskrete Logikbauelemente lassen sich energiesparend bei derart herabgesetzter Versorgungsspannung betreiben, daß zwar keine Veränderungen vorgenommen werden können, gespeicherte Signalzustände jedoch erhalten bleiben. Diese Bauelemente dienen der nachfolgenden Signalverarbeitung in dem Sinne, daß sie festlegen, ob auf ein Eingangssignal oder beim Erreichen eines vorgegebenen Zeitpunkts ein Stimulationsimpuls abgegeben werden soll.

Der von der Energiequelle 33, die im dargestellten Ausführungsbeispiel von einer Batterie gebildet wird, ausgehende Pfeil E deutet an, daß die außerhalb der strichpunktierten Linie angeordneten Bauelemente direkt von der Energiequelle 33 ohne Zwischenschaltung des Schaltelementes 32 versorgt werden.

Mit der dargestellten Anordnung sind die Betriebsfunktionen üblicher Schrittmachertypen mit synchroner oder asynchroner Stimulation im Atrium und/oder Ventrikel erzeugbar, wobei je nach Zustand der Schaltmittel 4 und Freigabe der entsprechenden Stimulationssignale bzw. Signalaufnahmemöglichkeit über die UND-Gatter 108 bis 110 bzw. 208 bis 210 auch jene Schrittmacherbetriebsarten höherer Ordnung möglich sind, wie sie in Form einer Übersicht beispielsweise in dem Aufsatz « Physiological Cardiac Pacing », R. Sutton, J. Perrins und P. Citron in « PACE » ; Vol. 3, März-April 1980, S. 207 bis 219 wiedergegeben sind.

Das grundsätzliche Stimulationsverhalten der verschiedenen Schrittmacherbetriebsweisen bei verschiedenartigen Herzsignalen ist ebenfalls in der vorgenannten Literaturstelle angegeben.

Weitere Einzelheiten zu den in der nachfolgenden Beschreibung dargestellten Schaltungsteilen können den am gleichen Tage eingereichten, diese Schaltungsteile wegen der damit verbundenen Neuerungen näher beschreibenden Patentanmeldungen WO 82/03781 bis 82/03786 derselben Anmelderin entnommen werden.

Durch die funktionsmäßige Trennung des komplexen, nur zeitweise aktivierten Logiksystems von den permanent betriebsbereiten Bauelementen, ist es in vorteilhafter Weise möglich, für den Fall des Ausfalls des umfangreichen Logikteils ein einfaches Ersatzsystem vorzusehen, durch welches ein Notbetrieb aufrecht erhalten werden kann. Obgleich zwar einerseits die Sicherheit einzelner Bauelemente gegen Ausfall in der Vergangenheit wesentlich gesteigert werden konnte, wird andererseits angestrebt, die Informationsverarbeitung in einem künstlichen Herzschrittmacher möglichst allen therapeutischen Anforderungen gerecht werden zu lassen, um dem Patienten eine optimale Stimulationstherapie zukommen zu lassen, und damit die erreichte Erhöhung der Zuverlässigkeit zum Teil wieder kompensiert.

Das dargestellte Konzept gestattet es, die Vorteile eines einfachen, höchst betriebssicheren Systems mit einem solchen höherer Verarbeitungsfähigkeit derart zu verbinden, daß bei Ausfall des komplexen Datenverarbeitungssystems ein einfaches Ersatzsystem bereitsteht, welches in der Lage ist, die Grundfunktionen des Schrittmachers unter nahezu allen Umständen für einen nur durch die Betriebsdauer der Energiequellen begrenzten Zeitraum sicherzustellen.

Diese Ersatzschaltung kann prinzipiell durch verschiedenartige Signale aktiviert werden. Eines dieser Signale ist ein von der komplexen Logikschaltung ausgehendes Signal « Par », das bei einer mittels eines Mikroprozessors realisierten Logikschaltung dann erscheint, wenn innerhalb der durchgeführten Operationen ein Plausibilitätsfehler ermittelt wurde, wie er beispielsweise durch eine Paritätsüberprüfung mit Hilfe von redundanten Signalen in bekannter Weise erzeugt werden kann. Entsprechende Signale lassen sich auch aus mit diskreten Logikbauelementen arbeitenden Schaltungen gewinnen, wenn hier zusätzliche Elemente verwendet werden, welche unzulässige Betreibszustände, beispielsweise in Form von Signalen, erkennen, die bei korrekt arbeitenden Logikmitteln beispielsweise nicht gleichzeitig oder aber nicht mit einer oberhalb oder unterhalb einer vorgegebenen Grenzfrequenz liegenden Rate etc. erscheinen dürfen. Derartige ein Fehlverhalten aufzeigenden Signale werden auch von den Ausgängen der Run-away-Schutzschaltungen 105 und 205 gewonnen und über ein ODER-Gatter 34 zusammengefaßt. Bei der dargestellten Anordnung kann es theoretisch in dem Fall zu einem Ansprechen der Run-away-Schutzschaltungen bei ordnungsgemäß arbeitenden Taktgenerator 2 kommen, wenn in die Speicher 121 oder 221 durch einen Fehler in dem durch die strichpunktierte Linie umrandeten Teil der Logikschaltungen fehlerhafte Zahlenwerte eingelesen wurden.

Dem ODER-Gatter 34 wird ein weiteres Signal M zugeführt, welches von einem Reed-Schalter stammt, der in der Zeichnung nicht dargestellt ist. Auf diese Weise ist es möglich, mittels eines von außen einwirkenden Magnetfeldes den Ersatzbetriebszustand entsprechend der « Magnetfrequenz » der bekannten Schrittmacher einzustellen.

Der Ausgang des ODER-Gatters 34 aktiviert eine Speicherschaltung 35, welche Zeitpunkten $T_A'$ und $T_V'$ entsprechende Zahlenwerte enthält, die bei Aktivierung dieser Speicherschaltung 35 mit Vorrang in die Speicher 121 bzw. 221 eingelesen werden. Gleichzeitig wird von der Schaltung 35 ein Ausgangssignal abgegeben, welches über invertierende Eingänge von UND-Gattern 111 und 211, die zwischen die Gatter 107 und 106 bzw. 207 und 206 eingeschaltet sind und diesen Signalweg sperren, während über die somit aktivierten UND-Gatter 112 und 212 eine direkte Verbindung zu den Ausgängen der Vergleicher 120 und 220 hergestellt wird.

Damit ist das im « DOO »-Betrieb arbeitende Ersatzsystem nicht mehr von der Signalverarbeitung der innerhalb der strichpunktierten Linie befindlichen Elemente der Schaltung abhängig. Die Werte $T_A'$ und $T_V'$ werden dabei so gewählt, daß sich eine für die Frequenzen annehmbare Ersatz-Stimulationsfrequenz ergibt. Die Betriebsart « DOO » bezieht sich auf den Anschluß zweier Elektroden. Ist jeweils nur eine der beiden Elektroden angeschlossen, so arbeitet der Schrittmacher im Zustand « AOO » oder « VOO » und gibt damit entsprechend der gewählten Elektrodenkonfiguration ebenfalls die zur Aufrechterhaltung der vitalen Funktionen notwendigen Stimulationsimpulse ab.

Mittels zweier in die Aktivierungsleitung des Schalters 222 eingefügter UND-Gatter 213 und 213a wird eine Synchronisation des Zählers 1 (und damit der Zeitsteuerung des Schrittmachers) auf vom Ventrikel erscheinende Signale für die Zeit der Überleitung vom Atrium zum Ventrikel verhindert (AV-Überleitung zwischen $T_A$ und $T_V$ in Figur 2), wenn gleichzeitig eine Signalaufnahme im Atrium möglich ist. Die entsprechenden Zeitmarken beziehen sich (abhängig von der Betriebsart des Schrittmachers) auf Zeiträume zwischen herzeigenen oder stimulierten Signalen. Dem UND-Gatter 213a werden das Signal $sens_A$ des Speichers 4 und das Signal « > » des Vergleichers 220 an seinen Eingängen zugeführt. Das Ausgangssignal des UND-Gatters 213a sperrt gegebenenfalls das UND-Gatter 213 über dessen invertierenden Eingang. Der weitere Eingang des UND-Gatters ist mit den die Signalaufnahme für das Atrium am UND-Gatter 110 freigebenden Signalausgang des Speichers 4 verbunden. Damit wird erreicht, daß eine Synchronisation bei regulärer Ventrikeltätigkeit stets nur mit $T_A$ erfolgt und somit das Atrium bei AV-sequentieller Stimulation die Führung behält bzw. bei fehlender Signalaufnahme aus dem Atrium der Abstand aufeinanderfolgender Zeitpunkte $T_A$ konstant bleibt.

Der hier beschriebene Schrittmacher stellt damit die physiologisch richtige Synchronisation mit dem Atrium sicher, wenn dort ungestörte Signale zur Verfügung stehen. Ist die Signalaufnahme im Atrium gestört, so erfolgt — entsprechend den vorgesehenen Möglichkeiten der Betriebszustandsänderungen, die weiter unten beschrieben werden — eine Umschaltung in einen eine Signalaufnahme im Ventrikel ermöglichenden Betriebszustand, so daß auch in diesem Fall eine korrekte Stimulation gewährleistet ist.

Obgleich bei freigegebener Signalaufnahme im Atrium für die Zeit t zwischen $T_A$ und $T_V$ keine Synchronisation des Zählers 1 durch im Ventrikel aufgenommene Signale erfolgt, wird die Refraktärzeit des Ventrikels dadurch in physiologisch sinnvoller weise gesetzt, daß diese sich trotz fehlender Synchronisation des Zählers 1 auf die Zeiten bezieht, zu denen die Herzaktion im Ventrikel festgestellt wurde, was bei dem dargestellten Ausführungsbeispiel dadurch realisiert ist, daß das Ausgangssignal des UND-Gatters 210 über ein UND-Gatter 214 und ein Schaltelement 215 einen Speicher 216 mit einem zu diesem Zeitpunkt im Zähler 1 enthaltenen Zahlenwert lädt, so daß dieser Zeitpunkt $T_V''$ als Bezugszeitpunkt jetzt für die Ermittlung der Refraktärzeit $T_{refV}$ durch den Vergleicher 7 dienen kann. Erfolgt eine Signalaufnahme ausschließlich im Ventrikel, so ist eine Synchronisation des Zählers durch das Schaltelement 222 im Zeitpunkt $T_V$ freigegeben, wodurch mittels des UND-Gatters 214 in den Speicher 216 dann derjenige Zahlenwert für $T_V$ übertragen wird, welcher auch im Speicher 221 vorhanden ist.

Nach Ablauf der Refraktärzeit für aus dem Ventrikel aufgenommene Signale erscheinende Impulse, welche von Extrasystolen herrühren, synchronisieren den Zeitzähler 1 des Schrittmachers, was bei dem dargestellten Ausführungsbeispiel dadurch erreicht wird, daß der Zähler 1 durch die Schaltermittel 222 auf den Wert $T_V$, der im Speicher 221 vorhanden ist, gesetzt wird. Darüberhinaus muß auch sichergestellt sein, daß retrograd übergeleitete Erregungen nach Extrasystolen keine Synchronisation des Schrittmachers bewirken. Dies wird dadurch erreicht, daß nach Erkennen der Extrasystole (Ventrikelaktivität vor dem Zeitpunkt $T_o$) die Atriumrefraktärzeit $t_{refA}$ für einen Zyklus auf 400 ms gesetzt wird, so daß für einen ausreichend langen Zeitraum (entsprechend der retrograden Überleitungszeit) — ausgehend von der Zeitmarke $T_V$ das Atrium für eine Signalaufnahme gesperrt ist.

Die Sperrung derartiger retrograder Überleitungen ist deshalb von besonderer Bedeutung, weil ein vom Schrittmacher auf das Signal im Atrium hin mit der vorgegebenen AV-Überleitungszeit abgegebener Stimulationsimpuls im Ventrikel eine Stimulation in der vulnerablen Phase zur Folge hatte.

Um hier den notwendigen Schutz zu bewirken, werden unmittelbar auf eine Extrasystole hin die Signalaufnahmemittel für Vorhof und Ventrikel für einen vorgegebenen Zeitraum refraktär geschaltet. Und zwar wird bei einem Eingangsimpuls am UND-Gatter 217 vom Ausgang der Eingangsstufe 201 für den Ventrikel, der erscheint, wenn auch der Vergleicher 220 am Ausgang « > » ein Signal abgibt, das an den zweiten Eingang des UND-Gatters 217 gelangt, ein Monoflop 218 gestartet. Dieses Monoflop gibt daraufhin einen Ausgangsimpuls von ca. 400 ms Dauer ab, welcher an einen Eingang des ODER-Gatter 219 gelangt, dessen zweiter Eingang mit dem Ausgang des Vergleichers 5 für die Refraktärzeit $T_{refA}$ verbunden ist, und dessen Ausgangssignal zu einem der Eingänge des UND-Gatters 110 übertragen wird.

Auf diese Weise ist sichergestellt, daß bei jeder nach der Zeit $T_V$ festgestellten Kammerkontraktion die Refraktärzeit für das Atrium neu gestartet und für die Zeitdauer des von dem Monoflop 218 abgegebenen Impulses (vorzugsweise 400 ms) aufrechterhalten wird, so daß eine Synchronisation des Schrittmachers durch retrograde Überleitungen verhindert ist. (Wie weiter unten näher erläutert ist, sind für diesen Zeitraum auch die Stör- und Tachykardieerkennungsmittel gesperrt, da diese bei einer retrograden Überleitung nicht ansprechen sollten. Die erforderliche Signalverbindung wird durch die Leitung vom Ausgang des Monoflops 218 zum Block 3 gebildet.) Ein ODER-Gatter sperrt auch den Ventrikel, wobei bevorzugt mittels — nicht dargestellter Schaltmittel — statt der Impulszeit des Monoflops 218 auch die in Block 7 enthaltene Zeit heranziehbar ist.

Für den Fall, daß gleichzeitig Signale im Atrium und Ventrikel aufgenommen werden, hat der Ventrikel Vorrang. Damit ist gewährleistet, daß die Sicherheit gegen schädliche Stimulation aufgrund unerwünschter Überleitungen auch bei nicht eindeutiger Signalerkennung zunächst einmal gegeben ist.

Gemäß einer bevorzugten Ausführung der Erfindung werden alle Umschaltungen (Programmänderungen, Betriebsartänderungen) zu Zeitpunkten vorgenommen, welche den Betriebsablauf nicht stören, das sind solche Zeitpunkte, zu denen keine Stimulation bewirkt werden kann und auch kein Eingangssignal erwartet wird, dessen Signalaufnahme wiederum von den eingestellten Betriebsparametern abhängig sein könnte. Ein derartiger Zeitpunkt ist derjenige auf $T_V$ folgende Zeitbereich, in dem beide Eingangsstufen refraktär sind (vergleiche Fig. 2, aus der die Zeiten des Herzzyklus im Hinblick auf den Betrieb des Schrittmachers in ihrer grundsätzlichen Verteilung ersichtlich sind).

Der mit $T_V$ beginnende Zeitraum ermöglicht dabei die für den Betrieb des Schrittmachers notwendigen Umschaltungen in einer Weise, welche die physiologischen Randbedingungen der Stimulation am wenigsten beeinträchtigen, da Umschaltungen zu dieser Zeit in eine natürliche Ruheperiode des Herzens fallen. Im Zeitpunkt $T_V$ finden auch solche Überprüfungen statt, die regelmäßig durchgeführt werden müssen, wie die Bestimmung des Betriebszustands der Energiequelle (EOL-Block 36).

Der Zeitpunkt $T_V$ wird bei normalen Herzverhalten stets durchlaufen und wäre an sich ausreichend beispielsweise die Übernahme der geänderten Betriebsparameter (entsprechender Pfeil an Speicherblock 4) oder die Änderung der Programmierung aufgrund der von außen her übertragenen entsprechenden Signale zu diesem Zeitpunkt zu bewirken (Pfeil nach Block 13).

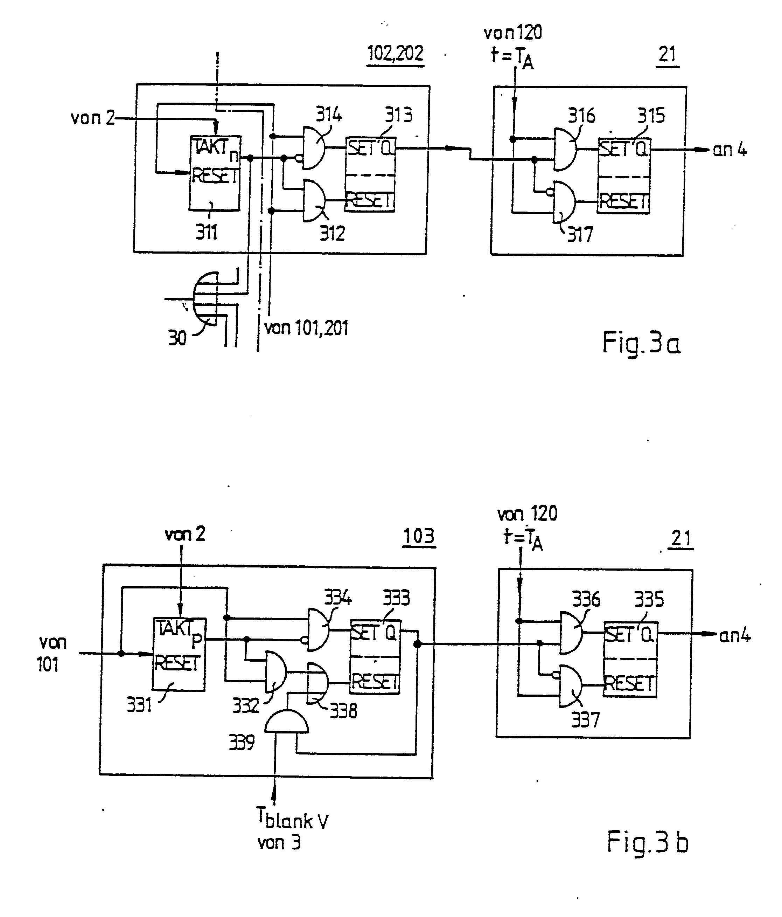

Die in Figur 1 mit den Blöcken 21, 102, 103, 202 dargestellten Schaltungen zur Ermittlung von Störsignalen im Vorhof oder Ventrikel bzw. zur Feststellung des Tachykardiezustandes sind in den Figuren 3a und b im einzelnen dargestellt. Dabei zeigt Figur 3a zum einen (in ihrem linken Bereich) die Detailschaltung der Bausteine 102 bzw. 202 gemäß Figur 1 und in ihrem rechten Teil eine Schaltung, wie sie einen Teil des Schaltungsblocks 21 in Figur 1 bildet — und zwar ist dieser Teil für die Verarbeitung der Eingangssignale von Atrium und Ventrikel je einmal vorhanden. Ein Verzögerungsglied 39 bewirkt, daß eine Umprogrammierung des Schrittmachers im Zeitpunkt $T_V$ erst dann erfolgt, wenn alle anderen bei dieser Zeitmarke auszuführenden Funktionen ausgeführt sind.

Die an den Eingang der Schaltung in Figur 3a gelangenden, von den Eingangsstufen 101 bzw. 201 in Figur 1 stammenden Impulse setzen einen Zähler 311 zurück, welcher durch Taktsignale des Taktgebers 2 (Figur 1) angesteuert wird. Erreicht der Zähler 311 einen Zählerstand n, so gibt er ein Ausgangssignal ab. Die Zahl n ist dabei so gewählt, daß die durch den Zählerstand n erzeugte Frequenzteilung des Eingangstaktes eine Ausgangsfrequenz erzeugen würde, welche gleich der oberen Grenzfrequenz der vom Schrittmacher zugelassenen Eingangssignale ist. Eingangsimpulse, welche eine höhere Rate aufweisen, erzeugen am Ausgang der Blöcke 102 bzw. 202 einen konstanten Pegel, den den Störzustand anzeigt.

Durch das Ausgangssignal n des Zählers 311 wird über ein UND-Gatter 312 ein nachgeschaltetes Flip-Flop 313 zurückgesetzt, wenn an dem anderen Eingang des UND-Gatters 312 der Impuls erscheint, welcher den Zähler 311 zurücksetzt. (Dabei wird davon ausgegangen, daß der Zähler nicht über den Stand n hinauszählt bzw. das Signal am Ausgang n beibehält, solange er nicht zurückgesetzt wird. Wird der Zählerstand n nicht erreicht, wenn der nachfolgende Impuls an dessen Eingang den Zähler zurücksetzt, so wird über ein weiteres UND-Gatter 314 das Flip-Flop 313 gesetzt, wobei der Ausgang « n » des Zählers mit einem invertierenden Eingang des UND-Gatters 314 verbunden ist. Eingangsimpulse, welche die Grenzrate zu Störsignalen überschreiten, setzen demnach das Flip-Flop 313, das vom nächsten Impuls, der mit einem größeren Zeitabstand als es der Störrate entspricht erscheint, zurückgesetzt wird.

Der Schaltzustand des Flip-Flops 313 wird in ein Flip-Flop 315 durch einen zum Zeitpunkt $t = T_A$ erscheinenden Impuls übertragen, wobei das Ausgangssignal des Flip-Flops 313 an den Eingang eines UND-Gatters 316 und den invertierenden Eingang eines UND-Gatters 317 gelangt, welche mit dem Setz- bzw. Rücksetzeingang des Flip-Flops 315 verbunden sind. Während also das Auftreten von Störsignalen fortgesetzt ermittelt wird, erfolgt eine Übernahme in das Flip-Flop 315 zur weiteren Verarbeitung jeweils nur zum Zeitpunkt $T_A$. Die Übernahme zum Zeitpunkt $T_A$ garantiert, daß die Störung richtig erkannt wurde und somit eine durch Blankingperioden verursachte Fehlinterpretation verhindert ist. Eine möglicherweise resultierende Umschaltung der Betriebsart folgt dann im Zeitpunkt $T_V$, zu Beginn der Refraktärzeit des Ventrikels. Eine Störerkennung ist damit auch grundsätzlich während der Refraktärzeiten mit Ausnahme der Austastzeiten möglich.

Die in Figur 3a wiedergegebene strichpunktierte Linie deutet (entsprechend der Darstellung in Figur 1) an, daß der Zähler 311 bei einem Betrieb mit teilweise zur Energieersparnis in einen Wartezustand setzbaren Schaltungsteilen permanent in Betrieb bleibt, während die rechts von der strichpunktierten Linie befindlichen Elemente lediglich bedarfsweise aktiviert werden, wenn nämlich der Ausgang des Zählers ein Signal an das ODER-Gatter 30 in Figur 1 ausgibt, welches eine Beendigung des Wartezustands der übrigen Schaltglieder auslöst. Hiermit wird also ein Beispiel für einen Betrieb gegeben, bei dem ein intern im Schrittmacher erzeugtes Zeitsignal, welches den Ablauf einer Zeitspanne markiert, in der ein erwartetes Ereignis nicht eingetreten ist, die Signalverarbeitungsmittel entsprechend diesem Ergebnis verändert.

Bei der in Fig. 3b dargestellten Ausführung der zur Feststellung des Tachykardiezustands vorgesehenen Logik entsprechen die Bauelemente 331 bis 337 den mit 311 bis 315 bezeichneten in Fig. 3a, wobei die Bezifferung in derselben Folge vorgenommen worden ist. Der Zähler 331 zählt jedoch bis zu einer Zahl p, welche so gewählt ist, daß eine Impulsfolgezeit definiert wird, welche die Grenze zu einer Impulsrate im Atrium bildet, die einen Tachykardie-Zustand kennzeichnet. Die genannte Folgezeit beträgt dabei 350 ms.

Der Rücksetzeingang des Flip-Flops 333 ist jedoch nicht direkt mit dem Ausgang des UND-Gatters 332 verbunden, sondern es ist hier ein ODER-Gatter 338 zwischengeschaltet, dessen weiterer Eingang mit dem Ausgang eines UND-Gatters 339 verbunden ist, dessen einer Eingang wiederum an den Ausgang des Flip-Flops 333 angeschlossen ist. Der andere Eingang des UND-Gatters 339 ist an den Ausgang der Austast-Schaltung 3 angeschlossen und erhält ein Signal, wenn auf einen Stimulationsimpuls hin die Atriumeingangsstufe 101 für Signale gesperrt wird. Damit wird erreicht, daß ein Rücksetzen des Flip-Flops 333 jeweils dann vorgenommen wird, wenn ein Stimulationsimpuls abgegeben wurde und eine Austastung der Atriumseingangsstufe 101 erfolgte. Auf diese Weise ist sichergestellt, daß das den Tachykardie-Zustand kennzeichnende Flip-Flop 335 stets erst dann zurückgesetzt wird, wenn nach einer Austastzeit der volle Zeitraum durchlaufen wurde, welcher zwischen zwei aufgenommenen Impulsen im Atrium liegen muß, um das Ende einer Tachykardie festzustellen. Damit können nicht durch die vorzeitige falsche Entscheidung der Annahme der Beendigung des Vorliegens einer Tachykardie Stimulationsimpulse zu Zeiten abgegeben werden, welche eine Gefährdung des Patienten bedeuten können.

Durch die gewählte Übernahme im Zeitpunkt $T_A$ über die weiteren Logikmittel (Betriebsartspeicher 4) ist weiterhin sichergestellt, daß die Veränderung des Betriebsverhaltens ebenfalls zu einem Zeitpunkt und in einer Weise erfolgt, welche eine optimale Synchronität mit dem Herzverhalten des Patienten sicherstellen.

Die von der Refraktärzeitsteuerung getrennte Ermittlung der Störungs- und Tachykardiezustände im Vorhof gewährleistet, daß beide Signalzustände optimal erfaßt werden, wobei durch das Zurücksetzen des Tachykardiezählers allein während der Austastzeiten sichergestellt ist, daß die Störerkennung unabhängig davon fortgesetzt wird und nicht durch ein Zurücksetzen und die daraus resultierende Bildung eines verkürzten Meßzeitintervalls für die Störerkennung im Zusammenhang mit den Austast-Impulsen ein Störsignal vorgetäuscht wird.

**Patentansprüche**

1. Herzschrittmacher, der die folgenden Merkmale aufweist :

a) erste Schaltmittel, welche auf vom Herzen abgeleitete Signale hin ein erstes Ausgangssignal erzeugen,

b) zweite Schaltmittel, welche Zeitgebermittel zur Festlegung eines Zeitpunktes, zu dem ein Stimulationsimpuls abzugeben ist, enthalten, wobei die Schaltmittel bei Erreichen des Zeitpunkts ein zweites Ausgangssignal erzeugen,

c) den ersten und zweiten Schaltmitteln nachgeschaltete Signalverarbeitungsmittel, denen das erste und zweite Ausgangssignal zugeführt werden, die eine Impulserzeugerschaltung zur Abgabe von Stimulationsimpulsen ansteuern, wobei die nachgeschalteten Signalverarbeitungsmittel von einem Wartezustand ohne oder mit nur geringem Energieverbrauch in einen Betriebszustand zur Signalverarbeitung setzbar sind, dadurch gekennzeichnet, daß dritte Schaltmittel (30, 31) vorgesehen sind, denen sowohl die von den ersten (101, 102) als auch von den zweiten Schaltmitteln (120, 220, 121, 221) erzeugten Ausgangssignale zugeführt sind, um auf das Erzeugen eines Ausgangssignals hin die nachgeschalteten Signalverarbeitungsmittel (von strichpunktierter Linie abgegrenzter Bereich in Fig. 1) für einen vorgegebenen Zeitraum vom Wartezustand in den Betriebszustand zu setzen, woraufhin die Signalverarbeitungsmittel die Zeitgebermittel der zweiten Schaltmittel zur Abgabe eines weiteren zweiten Ausgangssignals zu dem Zeitpunkt ($T_A$, $T_V$) voreinstellen, zu dem ein Stimulationsimpuls abzugeben ist und/oder die Impulserzeugerschaltung zur sofortigen Abgabe eines Stimulationsimpulses ansteuern.

2. Herzschrittmacher nach Anspruch 1, dadurch gekennzeichnet, daß bei programmierbare Logikschaltungen enthaltenden Signalverarbeitungsmitteln der Zustand mit geringem Energieverbrauch durch einen Standby-Zustand der zentralen Einheit (CPU) oder eine Inhibierung des Taktsignals einschaltbar ist.

3. Herzschrittmacher nach einem der Ansprüche 1 oder 2, dadurch gekennzeichnet, daß vorzugsweise bei diskrete Logikschaltungen enthaltenden Signalverarbeitungsmitteln der Zustand ohne oder mit geringem Energieverbrauch durch Abschaltung bzw. Herabsetzung der Versorgungsspannung (33) auf einen Spannungspegel einschaltbar ist, bei dem die Erhaltung des Speicherzustands für festzuhaltende Signalzustände noch gewährleistet ist.

4. Herzschrittmacher nach einem der vorangehenden Ansprüche, dadurch gekennzeichnet, daß zweite Zeitgebermittel (35) vorgesehen sind, die im Falle einer Störung auf ein entsprechendes Signal hin die Impulserzeugerschaltung zur Abgabe von Stimulationsimpulsen ansteuern.

5. Herzschrittmacher nach Anspruch 4, dadurch gekennzeichnet, daß das Signal das Ausgangssignal einer Prüfschaltung für die von den Signalverarbeitungsmitteln getroffenen logischen Entscheidungen bei Erkennung eines Fehlers bildet.

6. Herzschrittmacher nach Anspruch 4, dadurch gekennzeichnet, daß das Signal das Ausgangssignal von Prüfschaltungen für Paritäts-Bits der programmierbaren Logikschaltungen bildet.

7. Herzschrittmacher nach Anspruch 4, dadurch gekennzeichnet, daß das Signal das Ausgangssignal einer Run-Away-Schutzschaltung (105, 205) bildet, welche bei Überschreitung einer vorgegebenen Stimulationsimpulsrate ausgelöst wird.

8. Herzschrittmacher nach einem der Ansprüche 4 bis 7, dadurch gekennzeichnet, daß die Erzeugung von Stimulationsimpulsen auf das Signal hin in festfrequentem Betrieb erfolgt.

9. Herzschrittmacher nach Anspruch 8, dadurch gekennzeichnet, daß den Signalverarbeitungsmitteln die Ausgangssignale von Störerkennungsmitteln (102, 202) oder von Mitteln (103) zur Erkennung von Tachykardien zugeführt werden.

**Claims**

1. A cardiac pacemaker which comprises the following features :

a) first circuit elements which generates a first output signal in response to signals derived from the heart,

b) second circuit elements which contain timing means to determine a moment at which a stimulation impulse has to be delivered, the circuit elements generating a second output signal when the moment is reached,

c) signal processing means connected to the first and second circuit elements and to which the first and second output signals are supplied, which actuate an impulse generating circuit for the delivery of stimulation impulses, the signal processing means being able to be set from a waiting state without any or with only little energy consumption into an operating state for the signal processing,

wherein third circuit elements (30, 31) are provided to which the output signals produced both by the first circuit elements (101, 102) and by the second circuit elements (120, 220, 121, 221) are supplied in order to set the signal processing means (region defined by chain line in Figure 1) from the waiting state into the operating state for a preset period of time in response to the generation of an output signal, whereupon the signal processing means preset the timing means of the second circuit elements for the delivery of a further, second output signal at the moment ($T_A$, $T_V$) at which a stimulation impulse has to be delivered and/or activate the impulse generating circuit for the immediate delivery of a stimulation impulse.

2. A cardiac pacemaker as claimed in Claim 1, wherein with signal processing means containing programmable logic circuits, the state of low energy consumption can be switched on by a stand-by state of the central unit (CPU) or an inhibition of the clock signal.

3. A cardiac pacemaker as claimed in one of the Claims 1 or 2, wherein preferably with signal processing means containing discrete logic circuits, the state without any or with little energy consumption can be switched on by switching off or reducing the supply voltage (33) to a voltage level at which the maintenance of the store state for signal states to be retained is still ensured.

4. A cardiac pacemaker as claimed in one of the preceding Claims, wherein second timing means (35) are provided which, in the event of a disturbance, in response to a corresponding signal activate the impulse generating circuit for the delivery of stimulation impulses.

5. A cardiac pacemaker as claimed in Claim 4, wherein the output signal of a test circuit for the logical decisions reached by the signal processing means on detection of an error forms the signal.

6. A cardiac pacemaker as claimed in Claim 4, wherein the output signal from test circuits for parity bits of the programmable logic circuits forms the signal.

7. A cardiac pacemaker as claimed in Claim 4, wherein the output signal of a run-away protection circuit (105, 205), which is released if a preset stimulation impulse rate is exceeded, forms the signal.

8. A cardiac pacemaker as claimed in one of the Claims 4 to 7, wherein the generation of stimulation impulses in response to the signal is effected in fixed-frequency operation.

9. A cardiac pacemaker as claimed in Claim 8, wherein the output signals from disturbance detecting means (102, 202) or from means (103) for detecting tachycardias are supplied to the signal processing means.

**Revendications**

1. Stimulateur cardiaque comportant :

a) des premiers moyens de commutation qui génèrent un premier signal de sortie à partir de signaux dérivés du cœur ;

b) des deuxièmes moyens de commutation qui contiennent des moyens d'horloge pour fixer un instant où une impulsion de stimulation est à délivrer et qui génèrent un second signal de sortie lorsque cet instant est atteint ; et

c) des moyens de traitement de signaux, montés à la suite des premiers et des deuxièmes moyens de commutation et auxquels sont appliqués le premier et le second signal de sortie, qui attaquent un circuit générateur d'impulsions en vue de la délivrance d'impulsions de stimulation, les moyens de traitement de signaux étant susceptibles d'être amenés d'un état d'attente, à consommation d'énergie nulle ou faible, à un état de fonctionnement pour le traitement de signaux, caractérisé en ce que, il comporte des troisièmes moyens de commutation (30, 31), auxquels sont appliqués à la fois les signaux de sortie générés par les premiers (101, 102) et par les deuxièmes moyens de commutation (120, 220, 121, 221), pour, à la suite de la génération d'un signal de sortie, amener les moyens de traitement de signaux (la partie délimitée par la ligne en traits mixtes sur la figure 1) pour un intervalle de temps préfixé de l'état d'attente à l'état de fonctionnement, les moyens de traitement de signaux produisant ensuite le préréglage des moyens d'horloge des deuxièmes moyens de commutation en vue de la délivrance d'un second signal de sortie supplémentaire à l'instant ($T_A$, $T_V$) où une impulsion de stimulation est à délivrer et/ou l'attaque du circuit générateur d'impulsions en vue de la délivrance immédiate d'une impulsion de stimulation.

2. Stimulateur selon la revendication 1, caractérisé en ce que, au cas où les moyens de traitement de signaux contiennent des circuits logiques programmables, l'état à faible consommation d'énergie peut être enclenché par un état d'attente de l'unité centrale (CPU) ou par une inhibition du signal d'horloge.

3. Stimulateur selon la revendication 1 ou 2, caractérisé en ce que, de préférence dans le cas où les moyens de traitement de signaux contiennent des circuits logiques discrets, l'état à consommation d'énergie nulle ou faible peut être enclenché par la coupure de la tension d'alimentation ou par la réduction de la tension d'alimentation (33) à un niveau de tension où la conservation en mémoire des états de signal à retenir est encore assurée.

4. Stimulateur selon une des revendications précédentes, caractérisé en ce qu'il comporte des seconds moyens d'horloge (35) qui, à la suite d'un signal correspondant, attaquent le circuit générateur d'impulsions en vue de la délivrance d'impulsions de stimulation dans le cas d'une perturbation.

5. Stimulateur selon la revendication 4, caractérisé en ce que ledit signal constitue le signal de sortie d'un circuit de contrôle des décisions logiques prises par les moyens de traitement de signaux en cas de détection d'une erreur.

6. Stimulateur selon la revendication 4, caractérisé en ce que ledit signal constitue le signal de sortie de circuits de contrôle pour des bits de parité des circuits logiques programmables.

7. Stimulateur selon la revendication 4, caractérisé en ce que ledit signal constitue le signal de sortie d'un circuit de protection contre l'emballement (105, 205), le signal étant déclenché en cas de dépassement d'une fréquence préfixée des impulsions de stimulation.

8. Stimulateur selon une des revendications 4 à 7, caractérisé en ce que, à la suite dudit signal, les impulsions de stimulation sont générées dans un mode à fréquence fixe.

9. Stimulateur selon la revendication 8, caractérisé en ce que les moyens de traitement de signaux reçoivent les signaux de sortie de moyens de détection de perturbations (102, 202) ou de moyens (103) pour la détection de tachycardies.

Fig. 1

Fig. 2

Fig.3a

Fig.3 b